# EUROPÄISCHE PATENTSCHRIFT

(11) **EP 0 934 031 B1**
(45) Veröffentlichungstag und Bekanntmachung des Hinweises auf die Patenterteilung: **18.09.2002**
(21) Anmeldenummer: 97912216.5
(22) Anmeldetag: 24.10.1997
(51) Int. Cl.: A61M 1/36

(54) **VERFAHREN ZUM BETREIBEN EINER BLUT-ZENTRIFUGIEREINHEIT, SOWIE ZENTRIFUGIEREINHEIT ZUM DURCHFÜHREN EINES SOLCHEN VERFAHRENS**
PROCESS FOR OPERATING A BLOOD CENTRIFUGATION UNIT, AND CENTRIFUGATION UNIT FOR CARRYING OUT THE PROCESS
PROCEDE D'EXPLOITATION D'UNE UNITE DE CENTRIFUGATION DU SANG, ET UNITE DE CENTRIFUGATION DU SANG POUR METTRE EN OEUVRE CE PROCEDE

(30) Priorität: 25.10.1996 DE 19644336
(43) Veröffentlichungstag der Anmeldung: 11.08.1999
(73) Patentinhaber: Geigle, Peter, 63755 Alzenau (DE)
(72) Erfinder: Geigle, Peter, 63755 Alzenau (DE)
(74) Vertreter: Grimm, Ekkehard, Dipl.-Phys.
(86) Internationale Anmeldenummer: EP9705865
(87) Internationale Veröffentlichungsnummer: WO98018403

(56) Entgegenhaltungen:
- EP-A- 0 664 160
- WO-A-94/05349
- DE-C- 4 227 695

## Beschreibung

Die Erfindung beschreibt ein Verfahren zum Betreiben einer Zentrifugiereinheit, mit einem Zentrifugierbehälter, oder mit einer Zentrifugierglocke, gemäß Anspruch 1.

Weiterhin betrifft die Erfindung eine Zentrifugiereinheit, mit einem Zentrifugierbehälter, oder mit einer Zentrifugierglocke, gemäß Anspruch 8.

Um Bestandteile einer Flüssigkeit mit unterschiedlichem spezifischen Gewicht zu separieren, ist es üblich, Zentrifugiereinheiten einzusetzen. Solche Zentrifugiereinheiten, insbesondere Zentrifugierglocken, finden beim Separieren von Blut, um Blut in seine Bestandteile zu trennen, Anwendung. Blut wird, nach dem Spenden, üblicherweise in seine Hauptbestandteile, d.h. das Blutplasma, niedermolekulare Substanzen und Proteine und die zellularen Elemente, von denen die roten Blutkörperchen den wesentlichen Teil ausmachen, zerlegt.

Es ist bekannt, daß das Zentrifugieren von Blut chargenweise mittels einer Zentrifugierglocke erfolgt, die ein Aufnahmevolumen von einigen Hundert Millilitern besitzt. Für jeden Zentrifugiervorgang wird diese Zentrifugierglocke mit Blut gefüllt, zentrifugiert, bis sich die Bestandteile des Bluts mit unterschiedlichem Gewicht in der Zentrifugierglocke voneinander getrennt und geschichtet haben, um danach, nach Anhalten der Zentrifugiereinheit, die einzelnen Bestandteile aus der Glocke zu entnehmen.

Durch eine solche herkömmliche Zentrifugation kann bei sich drehender Glocke nur der Überlauf (d.h. die Komponente mit dem geringsten, spezifischen Gewicht) gewonnen werden, bis die Glocke mit schweren Bestandteilen gefüllt ist. Danach wird die Glocke gestoppt und im Stillstand werden die schweren Bestandteile aus der Glocke entfernt. Werden diese schweren Bestandteile benötigt (beim Zentrifugieren von Blut in der Regel die roten Blutkörperchen, Erythrozyten), stellt somit dieser Schritt die Gewinnung des Endprodukts dar. Das Anhalten der Glocke, das Entleeren sowie das Wiederbefüllen, um mehrere Liter Blut in seine Einzelbestandteile zu separieren, ist zeitaufwendig.

Eine Zentrifugiereinheit mit einer Zentrifugierglocke der Eingangs beschriebenen Art zum Zentrifugieren von Blut ist aus der US-A-3,145,713 bekannt. Die Zentrifugierglocke, die in den Figuren dieser Druckschrift gezeigt ist, weist in einer Ausführungsform einen zylindrischen oder konischen Behälter auf mit einem äußeren Ringraum, der zwischen der äußeren Behälterwand und einem inneren Einsatz gebildet ist. Über eine Drehdurchführung an der Oberseite des Zentrifugierbehälters wird, etwa in der Rotations-Achse des Behälters, Blut über einen Zulauf zugeführt. Von diesem Zulauf führt ein Kanal zu dem Boden des Zentrifugierbehälters, wo das noch nicht separierte Blut in den Ringraum abgegeben wird. Im oberen Bereich des Ringraums ist eine Verbindung mit einem Ablauf, der durch die Drehführung hindurchführt, über den die separierten, leichteren Bestandteile abgeführt werden, vorgesehen. Nachdem der Ringraum bzw. Separationsraum mit den schwereren, aus dem Blut separierten, roten Blutkörperchen gefüllt ist, wird der Zulauf unterbrochen und die Drehung der Zentrifugierglocke gestoppt. Dann werden die roten Blutkörperchen geeignet behandelt und anschließend in der Zentrifugierglocke tiefgefroren, um die gesamte Einheit, mit den roten Blutkörperchen tiefgefroren, zu bevorraten. Damit in dem Zentrifugierbehälter zwischen dem Zulauf und dem Ablauf ein Druckausgleich erfolgen kann, ist im oberen Bereich, d.h. im Bereich der Drehdurchführung, eine Verbindung zwischen dem Zulaufbereich und dem Ablaufbereich bzw. dem Separationsraum vorhanden.

Eine weitere Zentrifuge mit einem Zentrifugierbehälter bzw. einer Zentrifugierglocke ist aus der EP-B1-0 015 288 bekannt. Diese Druckschrift befaßt sich im wesentlichen mit der besonderen Ausbildung der Drehdurchführung, im Bereich derer der Zulauf und der Ablauf vorgesehen sind. Auch bei dieser Zentrifugierglocke wird der Zulauf über einen Zulaufkanal bis zum Bodenbereich der Glocke geführt, von wo aus das zugeführte Blut radial nach außen über einen radialen Kanal in einen ringförmigen Separationsraum überführt wird. Der Zulauf, im Bereich der Achse des Behälters angeordnet, ist von einem axialen Ringraum umgeben, der von dem Boden der Glocke bis zu seinem oberen Bereich führt, wo er mit dem Ablauf bzw. dem Separationsraum kurz geschlossen, d.h verbunden, ist. Diese Verbindung dient auch dazu, einen Druckausgleich zwischen den einzelnen Raumbereichen der Glocke zu erzielen.

Eine weitere Verfahrensweise zum Separieren von Blut in seine Bestandteile ist in der Europäischen Patentanmeldung EP-A1-0 014 093 offenbart. Die Vorrichtung zur Durchführung des Verfahrens umfaßt eine Zentrifuge mit einem Rotorkessel, in den, in radialer Richtung gesehen, zwei beutelartige Behälter übereinander gelegt werden. In den einen Behälter wird das noch nicht separierte Blut eingefüllt. Nach der Separation wird der Rotor angehalten und der zweite Behälter mit einer Flüssigkeit gefüllt, um einen ersten, aus dem Blut separierten Bestandteil in dem ersten Behälter unter dem Einströmen der Flüssigkeit in den zweiten Behälter herauszudrücken.

Wie der vorstehend beschriebene Stand der Technik zeigt, und wie bereits eingangs erläutert wurde, werden die Verfahren nach dem Stand der Technik chargenweise durchgeführt, d.h. es werden jeweils vorgegebene Mengen an Blut, die maximal dem Füllvolumen des Zentrifugenrotors entsprechen, behandelt, oder es wird das Volumen des Rotors ausgenutzt, um darin einen separierten Bestandteil, beispielsweise rote Blutkörperchen, anzusammeln, um dann den Separationsraum, nachdem er mit diesen roten Blutkörperchen gefüllt ist, zu entleeren oder aber diesen separierten Bestandteil direkt in der Zentrifugierglocke einzufrieren und aufzubewahren. Falls geringere Mengen, die unterhalb des Füllvolumens des Separationsraums eines solchen Zentrifugenrotors liegen, separiert sind, besteht die Gefahr, daß sich nach einem Anhalten des Rotors der separierte Bestandteil mit den anderen separierten Bestandteilen vermischt.

Es ist aus der vorstehenden Beschreibung ersichtlich, daß zum Separieren großer Mengen Flüssigkeit, die das Füllvolumen des Zentrifugierbehälters übersteigen, in einzelnen Chargen separiert werden muß, was ein mehrfaches Anlaufen und wieder Anhalten der Zentrifuge mit sich bringt und eine zeitaufwendige Verfahrensweise darstellt.

Da zur Trennung von Suspensionen mittels Trennglocken über den Schweregradienten, gerade im medizintechnischen, aber auch im biotechnischen Bereich, nur sehr geringe Schweregradientendifferenzen auftreten, werden die zu trennden Flüsigkeiten mit mehreren 1000 g (1g ist die einfache Erdbeschleunigung) beaufschlagt. Physikalisch gesehen bildet sich im Inneren der Trennglocke, durch deren Drehung, das gewünschte Schwerefeld aus. Auf jedes sich in der Trennglocke befindliche Teilchen wirkt somit eine Zentrifugalkraft F(Z) ein, die sich aus dem Produkt aus Dichte des Teilchens und der Zentrifugalbeschleunigung b(z) ergibt.

In die Zentrifugalbeschleunigung b(z) geht das Produkt aus dem Radius r und der Winkelgeschwindigkeit im Quadrat ein.

Klassische Glocken haben den Auslaß durchgehend über zwei eng zusammenstehende, statische Scheiben realisiert, die wie eine Pumpe oder wie eine Schälscheibe wirken, (siehe US-Patent 3,145,713, Spalte 5, Zeile 20 bis 32). Die sich im Betrieb ausbildende Flüssigkeitssäule wächst mit zunehmender Befüllung bis an die äußeren Ränder dieser Pumpe heran. Flüssigkeit, die die Scheiben berührt, wird abgebremst und nach innen geschleudert und kann somit aus dem Auslaß austreten.

Der Radius r dieser Scheiben liegt im Bereich von 2 cm. Somit bildet sich bei einer herkömmlichen, mit Flüssigkeit gefüllten Glocke eine zylindrische, innere Zone ebenfalls mit Radius r aus, die stets mit Luft gefüllt bleibt. Zwischen der Oberfläche der Flüssigkeitssäule und dem Drehpunkt der Trennglocke bildet sich somit eine Druckdifferenz aus, die sich als P=F/A berechnen läßt. A ist hierbei die Fläche.

Ausformuliert ergibt sich die Formel P=D*(2PI*r*n)². Hieraus leitet sich der Zusammenhang von Drehzahl, Radius und daraus resultierendem Druck ab, wie er aus der Grafik (1) ersichtlich wird.

Die meisten klassischen Glocken werden so lange durch den Einlaß am unteren Ende der Drehachse der Glocke gefüllt, bis z.B. die Separationskammer mit einer schweren Komponente - bei Blut z.B. die roten Blutkörperchen - gefüllt ist. Ist dies der Fall, so wird die Rotation der Glocke gestoppt, die Flüssigkeitssäule fällt zusammen und kann durch den Einlaß am Fußpunkt der Drehachse abgesaugt werden. Hier ist dann nur noch ein eventueller Höhenunterschied durch die Pumpe zu überwinden. Abgesaugtes Volumen in der Glocke wird durch Luftzufuhr durch den Auslaß ausgeglichen (siehe z.B. Offenlegungsschrift 27 45 041, Seite 15, 2. Absatz, 1. Satz ff.).

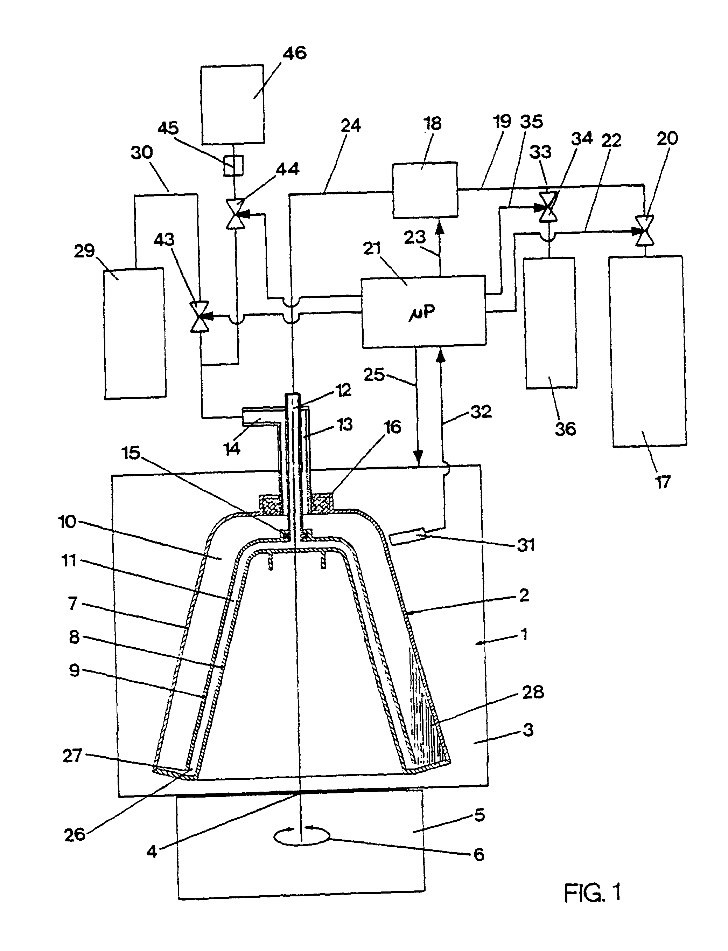

Neuere Glocken versuchen nun, das Abbremsen der Glocke zu vermeiden, indem versucht wird, die sich noch drehende Glocke zu leeren. Dies ist z.B. in dem US-Patent 4,879,031 beschrieben. Wie aus Figur 1 des US-Patents 4,879,031 hervorgeht, wird hierzu die zentrale Zuführung 11 gegen den zentralen Kanal 6 mit Dichtung 14 und 15 abgedichtet. Dann wird über den Einlaß 11b ein Unterdruck angelegt. Der anzulegende Unterdruck muß erheblich sein, da er die Flüssigkeit aus einer Entfernung, die sich aus der Drehachse (nicht dargestellt) und einer hierzu parallel verlaufenden Linie durch die äußere Umfangslinie der Scheiben 13a und 13b ergibt, ansaugen muß. Physikalisch ist ein Unterdruck auf 0 bar, dem absoluten Vakuum, also auf einen Unterdruck von 1 bar im Vergleich zur normalen Raumluft, begrenzt. Technisch endet ein erzeugbarer Unterdruck mit üblicherweise verwendeten Roller- oder Peristaltikpumpen bei ca. 200 mbar.

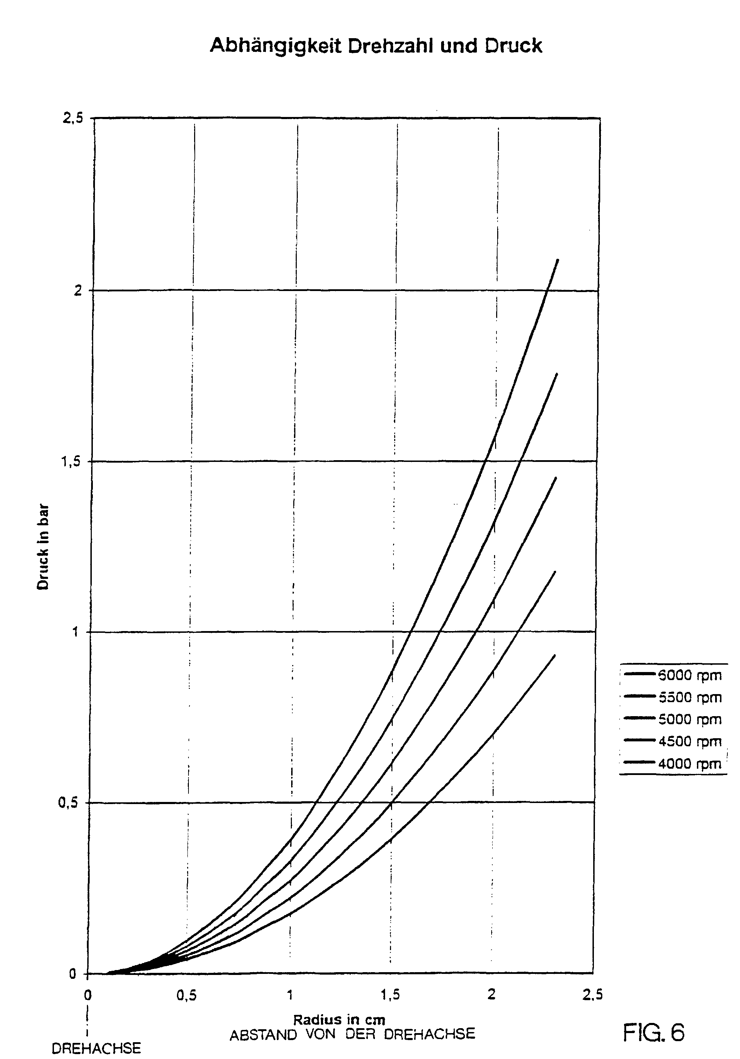

Aus dem Diagramm gemäß Figur 6, in dem der Druck (in bar) in Abhängigkeit von dem radialen Abstand von der Drehachse der Glocke aufgetragen ist, ist ersichtlich, daß bei den für diese Trennglocken üblichen Drehzahlen von 4.000 bis 6.000 rpm bereits ein Ansaugen der ersten Fraktions-Schicht an der physikalischen Grenze liegt. Keinesfalls ist es möglich, bei den oben genannten Drehzahlen Flüssigkeit aus radial weiter außen gelegenen Bereichen der Glocke abzusaugen. Ein Ausweg könnte das weitere Absenken der Drehzahl sein. Hierbei kommt es jedoch zu einer Auflösung der Trenngrenzen, was konträr zu Sinn und Zweck der Glocke dieses Patents wäre. Man hätte den gleichen Zustand wie bei einem vollständigen Anhalten der Glocke. Das US-Patent 4,859,333 hat die gleichen Ausgangsbedingungen, wie sie vorstehend angegeben sind. Auch hier erfordert das Absaugen das Anlegen eines sehr hohen Unterdrucks.

In der ersten Phase des Absaugens wird entsprechend der Verfahrensweise der US-PS 4,859,333 und der US-PS 4,879,031 zudem keine Flüssigkeit gefördert, sondern Luft, die sich in dem oben beschriebenen Maße im Zentrum befindet.

Auch ist allgemein bekannt, daß Zellen, und insbesondere rote Blutkörperchen, zwar gegen Druck sehr resistent sind, bei einem Anlegen von Unterdruck jedoch zur Haemolyse neigen.

Mit dem US-Patent 5,514,070 wird eine andere Möglichkeit gezeigt, Flüssigkeit aus einer sich drehenden Glocke abzusaugen. Wie in den Figuren zu sehen ist, wird am unteren Ende des Einlasses 26 eine zweite Pumpe, bestehend aus zwei Scheiben, mit denselben Radii wie die Scheiben 30 und 32, die am oberen Ende den klassischen Auslaß formen, vorgesehen. Ist nun die Separationskammer mit einer schweren Komponente - bei Blut z.B. die roten Blutkörperchen - gefüllt, so wird über den Auslaß 18 eine leichtere Komponente, hier z.B. Kochsalz, zugeführt (Spalte 2, Zeile 66 ff.). Hierdurch soll die schwerere Komponente durch den Durchlaß P2 gedrückt werden. Da ein "Hinterfüttern" einer schwereren Komponente mit einer leichteren Komponente nicht möglich ist, wird es, dem Prinzip der kommunizierenden Röhren folgend, zumindest zu einer Verdünnung kommen. Auch kommt es im Ringraum P3 zu einer steten Sedimentierung, die vom Sedimentationskoeffizient abhängig ist. Der Sedimentationskoeffizient s wird meist in Svedberg-Einheiten angegeben. Der Sedimentationskoeffizient s ist der Quotient aus der Wanderungsgeschwindigkeit von Teilchen in der Suspension und der Zentrifugalbeschleunigung. Es ergibt sich, daß die Wanderungsgeschwindigkeit direkt proportional zur Zentrifugalbeschleunigung ist. Dieser steht die Geschwindigkeit entgegen, mit der nun leichte Flüssigkeit durch den Auslaß 18 zugeführt wird. Sie steht in direktem Verhältnis zur Geschwindigkeit, mit der schwerere Zellen durch die Öffnung E1 in den Ringraum P3 strömen. Das Verhältnis wird durch die Fläche des Querschnitts des Auslasses zur Wandfläche des Ringraums P3 am geringsten. Am äußeren Durchmesser des Raums steht somit die höchste Sedimentationsgeschwindigkeit der geringsten, zum Durchlaß P2 gerichteten Strömungsgeschwindigkeit entgegen. Hierdurch kommt es, je nach Sedimentationskoeffizient, zu einer mehr oder minder starken Verdünnung.

Ausgehend von dem vorstehend beschriebenen Stand der Technik und der aufgezeigten Problematik, die sich immer dann ergibt, wenn Mengen an Flüssigkeiten separiert werden sollen, die das Füllvolumen des Separationsraums eines Zentrifugenrotors übersteigen, oder wenn das Füllvolumen des Separationsraums einen separierten Bestandteil übersteigt, liegt der vorliegenden Erfindung die Aufgabe zugrunde, ein Verfahren zum Betreiben einer Zentrifugiereinheit einerseits und eine Zentrifugiereinheit zum Durchführen eines solchen Verfahrens andererseits anzugeben, mit denen ermöglicht wird, auch größere Mengen an zu separierender Flüssigkeit in mindestens zwei Bestandteile zu zerlegen, ohne daß dazu die Zentrifuge chargenweise angehalten werden muß, wobei auch die Möglichkeit gegeben sein soll, in der Lage zu sein, ein Absaugen von den schwersten Bestandteilen bei voller Betriebsdrehzahl zwischen ca. 4.000 und ca. 6.000 rpm (Umdrehungen/Minute) ermöglichen. (Die Betriebsdrehzahl wird grundsätzlich nach unten durch ein Auflösen der Trenngrenzen und nach oben durch die mechanische Beanspruchbarkeit der Trennglocke begrenzt.) Beim Absaugen soll es zu einer möglichst geringen Verdünnung kommen.

Diese Aufgabe wird verfahrensgemäß durch die Merkmale des Anspruchs 1 gelöst.

In Bezug auf eine Zentrifugiereinheit der eingangs genannten Art wird die Aufgabe dadurch gelöst, daß der Zulauf, der Zufuhrkanal, der Separationsraum und der Ablauf in Strömungsrichtung gesehen aufeinanderfolgende, voneinander getrennte Strömungswege bilden und daß die Pumpeinrichtung, die Strömungsrichtung zwischen Zulauf und Ablauf umkehrend, umschaltbar ist zum Abführen, dann über den Zufuhrkanal und den Zulauf, eines separierten Bestandteils, wobei der Übergang zwischen dem Separationsraum und dem Ablauf nahe der Rotationsachse maximal 10 mm, vorzugsweise 2 bis 6 mm, von dieser beabstandet angeordnet ist.

Mit dem erfindungsgemäßen Verfahren ist es möglich, die separierten Bestandteile und die schweren Fraktionen der Zentrifugierglocke bzw. der Zentrifugiereinheit zu entnehmen, ohne daß die Drehung der Zentrifugiereinheit angehalten werden muß. Entsprechend dem erfindungsgemäßen Verfahren wird, nachdem eine ausreichende oder vorgegebene Menge an separierten Bestandteilen in dem Zentrifugierbehälter angereichert ist, die Strömungsrichtung, die zum Zuführen der noch nicht separierten Flüssigkeit eingehalten wurde, d.h. die Strömung zwischen dem Zulauf und dem Ablauf, umgekehrt, um dann über den Zulaufkanal, der definiert in einem Bereich des Separationsraums endet, einen separierten Bestandteil anzusaugen und über den eigentlichen Zulauf, der dann als Ablauf dient, im Bereich der Drehdurchführung der Zentrifugiereinheit nach außen abzuführen. Hierbei wird über den ursprünglichen Ablauf im Rahmen der Entnahme des separierten Bestandteils aus dem Separationsraum eine Flüssigkeit zugeführt, um das entnommene Volumen aufzufüllen. Bei dieser zugeführten Flüssigkeit kann es sich um eine artspezifische Flüssigkeit zu derjenigen Flüssigkeit handeln, die separiert wurde, d.h. es kann neue, noch nicht separierte Flüssigkeit über den Ablauf gleichzeitig mit der Entnahme des separierten Bestandteils nachgefüllt werden; vorzugsweise sollte, falls Blut separiert wird, Plasma oder eine andere leichte Flüssigkeit, zum Beispiel eine Kochsalz-Lösung, aufgefüllt werden. Nachdem der separierte Bestandteil aus dem Separationsraum vollständig entnommen ist, wird über den eigentlichen Zulauf, über den der separierte Bestandteil nun entnommen wurde, wieder zu separierende Flüssigkeit kontinuierlich zugeführt, bis der Separationsraum erneut mit den von der Flüssigkeit zu trennenden Bestandteilen angereichert ist. Danach wird wieder die Strömungsrichtung, wie vorstehend erwähnt, umgekehrt, so daß erneut eine Entnahme des separierten Bestandteils erfolgen kann. In Bezug auf die Zentrifugiereinheit ist es wesentlich, um die erfindungsgemäße Verfahrensweise vornehmen zu können, daß die Strömungswege zwischen dem Zulauf, dem Zulaufkanal, dem Separationsraum und dem Ablauf voneinander geströmte Strömungswege bilden, d.h. es ist nur eine definierte Strömung der Flüssigkeit von Bereich zu Bereich möglich. Eine solche Zentrifugiereinheit darf keine unmittelbaren strömungsmäßigen Verbindungen zwischen Zulauf und Ablauf besitzen, beispielsweise um irgendeinen Druckausgleich vorzunehmen, da ansonsten über solche Kurzschlußverbindungen bei der Umkehrung der Strömungsrichtung zwischen Zulauf und Ablauf beim Entnehmen eines separierten Bestandteils nachgeführte Flüssigkeit, um das entnommene Volumen aufzufüllen, unmittelbar zu dem Ablauf hin strömen kann und sich mit dem separierten Bestandteil vermischen kann. Vorzugsweise wird mit einem solchen Verfahren und einer solchen Zentrifugiereinheit gemäß der Erfindung ein separierter Bestandteil mit hohem spezifischen Gewicht aus der Zentrifuge entnommen. Es ist aber auch möglich, leichtere Bestandteile, die aus einer Flüssigkeit, beispielsweise Blut, separiert sind, dem Separationsraum mit der erfindungsgemäßen Verfahrensweise zu entnehmen. Hierzu muß der Zentrifugierbehälter in Bezug auf die Rotationsachse der Zentrifugiereinheit eine entsprechende Form aufweisen und der Zufuhrkanal, über den der separierte Bestandteil abgesaugt werden soll, muß in einer solchen Zone enden, in der sich der separierte, zu entnehmende Bestandteil der Flüssigkeit ansammelt.

Um Bestandteile aus der Flüssigkeit, die ein hohes spezifisches Gewicht besitzen, abzusaugen, wird in einer bevorzugten Verfahrensweise und einem bevorzugten Aufbau der Zentrifugiereinheit der Zufuhrkanal im Bodenbereich des Zentrifugierbehälters endend angeordnet. Die Zentrifugiereinheit kann dann so lange betrieben werden, bis im wesentlichen der gesamte Separationsraum mit dem spezifisch schweren Bestandteil der Flüssigkeit, die kontinuierlich über den Zufuhrkanal zugeführt wird, angereichert ist, während über den Ablaufkanal die leichteren Bestandteile abgeführt werden. Erst nachdem die ausreichende Menge angesammelt ist, wird die Strömungsrichtung wieder umgekehrt, um die schweren Bestandteile, allerdings unter weiterer, fortwährender Rotation des Zentrifugierbehälters, zuführen. Es wird ersichtlich werden, daß dadurch, daß sich die Zentrifugiereinheit ständig unter Rotation befindet, auch während der Entnahme des separierten Bestandteils, keine Vermischung mit anderen Bestandteilen, die ein anderes spezifisches Gewicht haben, die nicht unmittelbar abgeführt werden sollen, auftritt.

Hierzu sind folgende konstruktiven Details und Eigenschaften wesentlich:

Die Glocke bzw. der Zentrifugierbehälter soll sich auch bei voller Betriebsdrehzahl vollständig mit Flüssigkeit füllen. Hierzu muß die Öffnung des Auslasses direkt an der Wandung des Einlasses beginnen. In einer vollständig mit Flüssigkeit gefüllten Trennglocke bilden sich dann keine Druckdifferenzen heraus.

Desweiteren sollten Zulaufkanäle vorgesehen werden, die möglichst weit am unteren Durchmesser mit ihrer Öffnung in den Separationsraum münden. Diese Zulaufkanäle sollen im Durchmesser möglichst gering sein (ca. 2 bis 6 mm), um beim Absaugen eine hohe Strömungsgeschwindigkeit zu erreichen.

Verfahrenstechnisch ist darauf zu achten, daß die Glocke stets mit Flüssigkeit gefüllt bleibt, solange ein Absaugen bei voller Betriebsdrehzahl gewünscht wird. D.h., daß durch die Öffnung Zulaufkanals und den Auslaß abgesaugte Flüssigkeit oder Suspension gleichzeitig durch eine geeignete Flüssigkeit oder Suspension durch den anderen Zulauf bzw. Ablauf ersetzt werden muß. Hierdurch wird die Drucklosigkeit des Systems gewährleistet.

Die noch leere Glocke soll sich zu Beginn der Befüllung nicht oder nur sehr langsam drehen. Hierdurch wird eine vollständig Füllung der Zulaufkanäle erreicht. Zugleich wird der bei nicht vollständig gefüllter Glocke auftretende Sogeffekt einer Zentrifugalpumpe minimiert. Es kann jedoch auch gewünscht sein, diesen Sogeffekt gezielt einzusetzen, um z.B. aus Gefäßen zu trennende Flüssigkeit anzusaugen oder auch um Druckverluste in Schlauchsystemen zu kompensieren. Dies geschieht durch eine berechnende Anpassung der Drehzahl an den gewünschten Saugeffekt.

Ist die Glocke vollständig oder fast vollständig gefüllt, kann die gewünschte Betreibsdrehzahl erreicht werden. Die Suspension wird sich gemäß der unterschiedlichen Dichten aufteilen. Bei Blut wird sich z.B. zur Mitte hin Plasma sammeln, während sich die schweren roten Blutkörperchen nach außen bewegen. Zwischen diesen beiden Fraktionen werden sich die weißen Blutkörperchen und die Thrombozyten als dünne Schicht sammeln.

Bei einem weiteren Befüllen mit Blut wird Plasma durch einen Überlaufkanal und den Auslaß austreten. Das Befüllen der Glocke kann wahlweise mechanisch, z.B. durch eine Pumpe erfolgen oder mittels Schwerkraft z.B. direkt über eine Nadel mit geeignetem Schlauch von einem etwas höher als die Trennglocke positionierten Spender.

Um die Verfahrensweise zu automatisieren, wird eine Sensoreinrichtung vorgesehen, die einen bestimmten Füllstand in dem Zentrifugierbehälter eines zu separierenden und dann abzuführenden Bestandteils erfaßt, die dann einen Impuls auslöst, über den die Umschaltung der Pumpeinrichtung angesteuert wird. Vorzugsweise handelt es sich hierbei um eine optische Vorrichtung, die darüber hinaus außerhalb des Zentrifugierbehälters positioniert ist, sich also nicht mit diesem dreht. Nachdem ein solcher Füllstandsimpuls ausgelöst ist, kehrt die Pumpeinrichtung, die zunächst zu separierende Flüssigkeit über den Zulauf zugeführt hatte, ihre Pumprichtung um, um über den normalerweise als Zulauf dienenden Anschluß den separierten Bestandteil dem Zentrifugierbehälter zu entnehmen. Da das Volumen des Separationsraums eine bekannte Größe ist, kann die abzuführende Menge, d.h. die Menge des separierten Bestandteils, vorab eingestellt werden, so daß die Pumpeinrichtung, nach der Abführung eines vorgegebenen, definierten Volumens an separiertem Bestandteil, ihre Pumprichtung wieder umkehrt, um dann erneut zu separierende Flüssigkeit über den Zulauf zuzuführen.

Der Zulaufkanal der erfindungsgemäßen Zentrifugiereinheit wird bevorzugt in mindestens zwei Abschnitte unterteilt, wobei der erste Abschnitt mit seiner Achse entlang der Rotationsachse des Zentrifugierbehälters verläuft. Diese Achse stellt die neutrale Zone der Zentrifugiereinheit dar, so daß zum einen nicht bereits in dem Zulaufkanal eine Separation der noch nicht separierten Flüssigkeit erfolgt, zum anderen dadurch der Schwerpunkt der Zentrifugiereinheit auf der Rotationsachse verbleibt. Der zweite Abschnitt des Zulaufkanals kann dann zweigeteilt sein, d.h. es wird am Ende des ersten Abschnitts der zweite Abschnitt an gegenüberliegenden Seiten sich verzweigend radial nach außen geführt. Falls mehr als zwei Kanäle vorgesehen werden, sollte immer darauf geachtet werden, daß eine gewisse Symmetrie eingehalten wird, so daß keine Unwucht beim Rotieren der Zentrifugiereinheit auftritt. Sollte nur ein einzelner Zufuhrkanal bzw. ein einzelner, zweiter Abschnitt für den Zufuhrkanal vorgesehen sein, sollte ein entsprechender Gewichtsausgleich auf der radial gegenüberliegenden Seite erfolgen. Ein einzelner Zufuhrkanal hat den Vorteil, daß gerade beim Absaugen ein relativ kleiner Absaug-Querschnitt gegeben ist, um möglichst viel des separierten Bestandteils absaugen zu können, ohne daß weitere Bestandteile erfaßt werden.

Um das Volumen des Zentrifugierbehälters weitgehendst zur Separation zu nutzen, wird der Behälter so aufgebaut, daß der Separationsraum und der Zufuhrkanal durch eine gemeinsame Trennwand voneinander getrennt sind. Das bedeutet, daß kein weiterer Zwischenraum zwischen dem Zufuhrkanal und dem Separationsraum vorhanden ist. Während der Zufuhrkanal mit einem relativ kleinen Durchmesser dimensioniert wird, d.h. in der Größenordnung von einigen Millimetern Durchmesser, wird der gesamte, den Zufuhrkanal umgebende, radiale Bereich bis zur Außenseite des Behälters für den Separationsraum genutzt. Abhängig von den zu separierenden Bestandteilen sollte der Separationsraum eine spezifische Form und Orientierung zur Drehachse aufweisen. Hierbei ist es bevorzugt, daß der Separationsraum in seiner Erstreckung in Strömungsrichtung zu der Drehachse geneigt verläuft derart, daß das zu dem Ablauf hin liegende Ende näher zu der Drehachse als der Übergang zwischen dem Zufuhrkanal und dem Separationsraum liegt. Der Ablaufkanal ist hierbei derjenige Kanal, über den im Betrieb zum Separieren der Bestandteile diejenigen Bestandteile abgeführt werden, die nicht als separierte Bestandteile in dem Separationsraum während der Trennphase verbleiben.

Weitere Einzelheiten und Merkmale der Erfindung ergeben sich aus der nachfolgenden Beschreibung von Ausführungsbeispielen anhand der Zeichnung.

In der Zeichnung zeigt
- Figur 1: einen Längsschnitt entlang der Rotationsachse einer erfindungsgemäßen Zentrifugiereinheit mit Zentrifugierbehälter, wobei darüberhinaus schematisch die Pumpeinrichtung sowie die Sensoreinrichtung und eine dazugehörige Steuereinheit angedeutet sind,
- Figur 2: eine weitere Ausführungsform eines Zentrifugierbehälters,
- Figur 3: einen Schnitt entlang der Schnittlinie III-III in Figur 2,
- Figur 4: einen Längsschnitt einer dritten Ausführungsform eines Zentrifugierbehälters,
- Figur 5: einen Schnitt entlang der Schnittlinie V-V in Figur 3, und
- Figur 6: ein Diagramm, das den Druck, der auf ein Partikel in einem Zentrifugierbehälter einwirkt, in Abhängigkeit von dem Abstand von der Drehachse des Zentrifugierbehälters für fünf verschiedene Drehzahlen darstellt.

Die Anordnung, wie sie in Figur 1 schematisch dargestellt ist, umfaßt eine Zentrifugiereinheit 1 mit einem Zentrifugierbehälter 2 bzw. einer Zentrifugierglocke 2, die in einem Rotorraum 3 um eine Drehachse 4 mittels einer einen Motor umfassenden Antriebseinheit 5 drehbar ist, wie durch den Drehpfeil 6 angedeutet ist.

Die Zentrifugierglocke 2 besitzt eine kegelstumpfförmige äußere Form mit einem die äußere Behälterwand 7 bildenden Kegelstumpfmantel, einem die innere Behälterwand 8 bildenden weiteren Kegelstumpfmantel und einem kegelstumpfmantelförmigen Einsatzteil 9, das den Raum zwischen der äußeren Behälterwand 7 und der inneren Behälterwand 8 in zwei Raumabschnitte unterteilt. Der, im Querschnitt senkrecht zur Drehachse 4 gesehen, äußere Raum bildet den eigentlichen, ringförmig ausgebildeten Separationsraum 10, während der Teilraum zwischen dem Einsatzteil 9 und der inneren Behälterwand 8 einen Ringraum bildet, der als Zufuhrkanal 11 dient. Wie aus der Dimensionierung ersichtlich wird, weist der Separationsraum 10 senkrecht zur Drehachse 4 einen wesentlich größeren Querschnitt auf als der sehr schmal dimensionierte Zufuhrkanal 11.

An der Oberseite des Zentrifugierbehälters 1 ist ein mittlerer Zulaufkanal 12 vorgesehen, der mit seiner Achse entlang der Drehachse 4 des Rotors verläuft. Dieser Zulaufkanal 12 steht mit dem Zufuhrkanal 11 strömungsmäßig in Verbindung. Die Verbindungsstelle ist unmittelbar in den Bereich nahe der Rotationsachse bzw. der Drehachse 4 gelegt. Der Zulaufkanal 12 ist radial von einem zweiten Rohr, das einen Ablaufkanal 13 bildet, mit Abstand umgeben, das einen seitlichen Stutzen 14 besitzt. Die Verbindungsstelle zwischen dem Ablaufkanal und dem Separationsraum ist ebenfalls in den Bereich nahe der Drehachse 4 gelegt, was von Vorteil bei dem Abpumpen separierter Bestandteile unter voller Betriebsdrehzahl der Glocke, wie sich aus Figur 6 ergibt. Dieser geringe Abstand von der Drehachse 12 und Ablaufkanal 13 wird durch deren koaxiale Anordnung in Bezug auf die Drehachse 4 erreicht. Sowohl der Zulaufkanal 12 als auch der Ablaufkanal 13 bzw. die entsprechenden Anschlußrohre sind Drehdurchführungen, die unter Rotation des Zentrifugierbehälters 2 feststehen, allerdings ausreichend über jeweilige Radialdichtungen 15, 16 den Separationsraum 10 und den Zufuhrkanal 11 gegen die Außenumgebung abdichten.

Um eine Flüssigkeit in ihre Bestandteile, d.h. Bestandteile mit unterschiedlichem, spezifischen Gewicht, zu separieren, wird diese Flüssigkeit aus einem Vorratsbehälter 17 mittels einer Pumpe 18 angesaugt. In die Zufuhrleitung 19 ist ein elektromagnetisch betätigbares Absperrventil 20 eingebaut, das über einen Mikroprozessor 21, mit einer Steuerleitung 22 verbunden, ansteuerbar ist. Zum Betreiben der Zentrifugiereinheit wird über den Mikroprozessor 21 der Verfahrensablauf gestartet, indem die Pumpe 18, über eine Leitung 23, von dem Mikroprozessor 21 eingeschaltet wird, gleichzeitig wird das Ventil 20 geöffnet, so daß die Pumpe 18 aus dem Vorratsbehälter 17 die Flüssigkeit, beispielsweise Blut, entnimmt und über die Zufuhrleitung 24 den Zulaufkanal 12 und von dort dem Zufuhrkanal 11 zuführt. Die Zufuhrleitung 19 kann, falls die Vorrichtung zum Separieren von Blut eingesetzt wird, unmittelbar mit der Spender-Person verbunden werden, wobei dann der Vorratsbehälter 17 entfallen würde.

Zu Anfang des Zentrifugierverfahrens kann die Zentrifugierglocke stillstehen, es ist allerdings auch möglich, bereits die Zentrifuge, über den Mikroprozessor 21 und die Steuerleitung 25 angesteuert, unter der erforderlichen Drehzahl rotieren zu lassen.

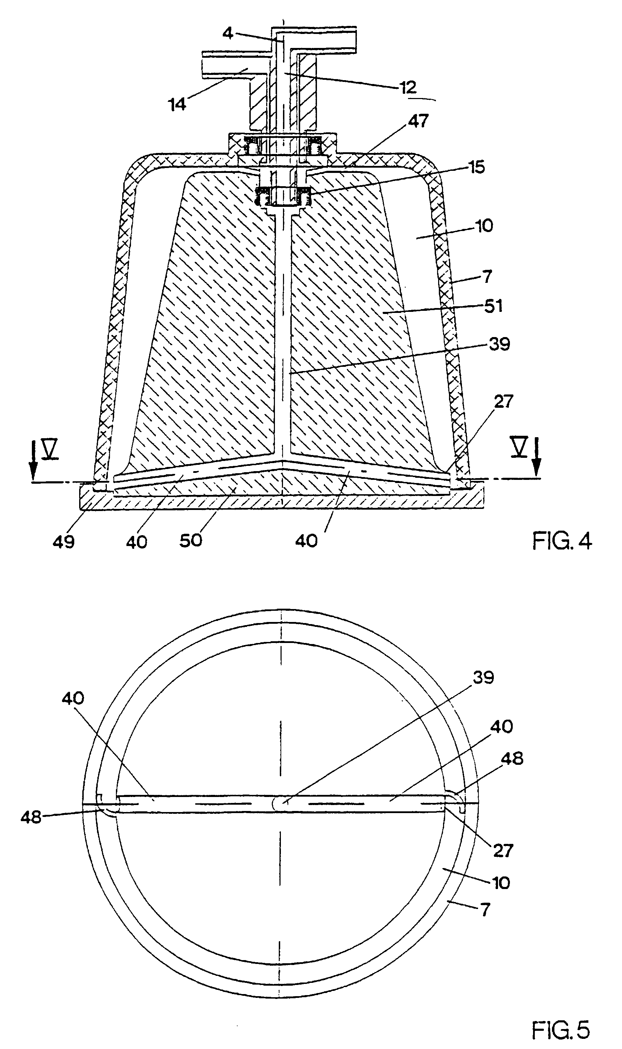

Die Flüssigkeit aus dem Vorratsbehälter 17 wird dann bis zu dem bodenseitigen Ende 26 des Zufuhrkanals 11 geführt, wo eine Öffnung oder Verbindungsstelle 27 zu dem Separationsraum 10 vorhanden ist. Über die Verbindungsstelle 27 tritt die zugeführte Flüssigkeit in den Separationsraum 10 ein, wo sich radial nach außen die einzelnen Bestandteile der zugeführten Flüssigkeit entsprechend ihrem spezifischen Gewicht schichten. Aufgrund der konischen bzw. kegelstumpfförmigen Form der Zentrifugierglocke 2 bzw. des Separationsraums 10 wird die Fraktion mit dem höchsten spezifischen Gewicht, die in der zugeführten Flüssigkeit vorhanden ist, im unteren, radial am weitesten nach außen liegenden Bereich des Separationsraums 10 der Zentrifugierglocke angesammelt, wie in Figur 1 in der unteren, linken Seite des Separationsraums 10 durch die schraffierte Fläche 28 angedeutet ist. Unter weiterer Zufuhr von zu separierender Flüssigkeit aus dem Vorratsbehälter 17 werden leichtere Bestandteile der Flüssigkeit, die sich näher zu der Rotationsachse 4 des Zentrifugierbehälters 2 ansammeln, über den Ablaufkanal 13 und den seitlichen Stutzen 14 herausgeführt und zu einem Sammelbehälter 29 über eine Ablaufleitung 30 geleitet. Die Verbindungsstelle 27 sollte möglichst weit radial nach außen zu der Behälterwand 7 gelegt werden, wie dies auch in der Ausführungsform des Zentrifugierbehälters, wie er in Figur 4 dargestellt ist, zu sehen ist.

Die vorstehend angegebene Verfahrensweise wird so lange, kontinuierlich, fortgeführt, bis der Separationsraum 10 der Zentrifugierglocke 2 mit der spezifisch schweren Fraktion der Flüssigkeit gefüllt ist. Eine definierte Menge, d.h. die Füllstandshöhe, der separierten Fraktion, beispielsweise roter Blutkörperchen, falls Blut in seine Bestandteile getrennt werden soll, wird über eine Sensoreinrichtung 31, bei der es sich um einen optischen Sensor handeln kann, erfaßt und über eine Sensorleitung 32 ein entsprechender Impuls an den Mikroprozessor 21 übermittelt. Bei der Sensoreinrichtung 31 kann es sich um einen optischen Detektor handeln, falls sich der aus der Flüssigkeit separierte Bestandteil, der in dem Separationsraum 10 verbleibt, optisch von den anderen Bestandteilen unterscheidet. Es ist aber auch möglich, andere geeignete Detektoren für diese Sensoreinrichtung 31 einzusetzen. Die Sensoreinrichtung 31 befindet sich außerhalb des Zentrifugierbehälters 2, jedoch dicht zu dessen Oberfläche zugeordnet. Üblicherweise wird ein solcher Zentrifugierbehälter bzw. eine solche Zentrifugierglocke 2 aus transparentem Kunststoffmaterial hergestellt. Um unterschiedliche Füllmengen in dem Rotor über diese Sensoreinrichtung 31 erfassen zu können, können geeignete Maßnahmen vorgesehen werden, um die Sensoreinrichtung 31 in radialer Richtung verschiebbar zu halten und sie in geeigneten Höhen oberhalb des bodenseitigen Endes 26 der Zentrifugierglocke 2 anzuordnen,

Nachdem der Mikroprozessor 21, über die Sensorleitung 32, eine entsprechende Information über das Erreichen einer bestimmten Zustandshöhe von der Sensoreinrichtung 32 erhalten hat, wird, unter voller Betriebsdrehzahl des Zentrifugierbehälters, die Zufuhr der zu separierenden Flüssigkeit von dem Vorratsbehälter 17 unterbrochen, indem die Pumpe 18 angehalten und/oder das Absperrventil 20 geschlossen wird. Danach wird die Pumprichtung der Pumpe 18 umgekehrt, so daß über den Zulaufkanal 12 nunmehr angesaugt wird, so daß der separierte Bestandteil, entsprechend der schraffierten Fläche 28, über die Verbindungsöffnung 27 in den Zulaufkanal 11 angesaugt und über die Leitung 24 entnommen wird. Diese Vorgänge werden unter weiterer, ständiger Rotation des Behälters durchgeführt. Das Ventil 20 kann in dieser Phase noch geöffnet verbleiben, so daß diejenige Flüssigkeit, die sich noch in dem Kanal 12, der Leitung 24, der Pumpe 18 und der Leitung 19 befindet, zurück in den Vorratsbehälter 27 gepumpt wird. Wenn der separierte Bestandteil eine Verzweigungsstelle 33 erreicht, wird das Absperrventil 20 über den Mikroprozessor 21 geschlossen und ein weiteres Absperrventil 34, das über eine Steuerleitung 35 mit dem Mikroprozessor 21 in Verbindung steht, wird geöffnet, so daß der separierte Bestandteil in einen Fraktionsbehälter 36 überführt wird. Die Entnahme wird so lange fortgeführt, bis der Separationsraum 10 von der separierten Fraktion entleert ist. Hierzu kann ein weiterer Sensor vorgesehen werden, der dem unteren Bereich des Separationsraums 10 zugeordnet ist. Eine andere Möglichkeit, die zu bevorzugen ist, besteht allerdings darin, daß unmittelbar in der Leitung 12 vor der Verzweigungsstelle 33 eine weitere, in Figur 1 nicht dargestellte, Sensoreinrichtung angeordnet wird, die erfaßt, wenn sich in der Leitung keine fraktionierten Bestandteile mehr befinden. Zu diesem Zeitpunkt wird, über den Mikroprozessor 21 gesteuert, das Absperrventil 34 wieder geschlossen, so daß sichergestellt ist, daß in den Fraktionsbehälter 36 nur der fraktionierte Anteil überführt wird.

Es ist ersichtlich, daß beim Absaugen des fraktionierten Bestandteils aus dem Separationsraum 10 Flüssigkeit aus dem Sammelbehälter 29 über die Ablaufleitung 30 zugeführt werden kann, um einen entsprechenden Fluidausgleich für die abgeführte Fraktion über den dann als Zulauf dienenden Ablaufkanal 13 zu schaffen. Es kann aber auch eine andere Flüssigkeit über einen weiteren, nicht dargestellten Behälter zugeführt werden.

Es muß ein Füssigkeits- bzw. Volumenausgleich stattfinden, damit die Glocke stets drucklos bleibt.

Durch ein gezieltes Entfernen oder Zuführen von leichten oder schweren Bestandteilen ist es grundsätzlich möglich, jede gewünschte Franktion der zu trennenden Flüssigkeit gezielt zu gewinnen oder auch in der Trennglocke anzureichern.

Wird eine Zentrifuge stehts mit gleicher Geschwindigkeit betrieben, kommt es zu keiner Geschwindigkeitsdifferenz zwischen der Trennkammer und der zu trennenden Flüssigkeit. Im vorliegenden Fall kann es somit beim Befüllen der Glocke im Bereich des Auslasses 27 zu einer eventuell ungewollten Kumulierung von noch unseparierter Flüssigkeit kommen. Um dies zu vermeiden, kann der Auslaß 27 so geformt werden, daß die austretende Flüssigkeit in der Trennglocke eine Sekundärbewegung erzeugt und so eine bessere Verteilung der zu separierenden Flüssigkeit über den Trennraum erreicht wird.

Auch kann durch Abbremsen und nachfolgendes Beschleunigen der Trennglocke um wenige rpm (U/min) eine solche bessere Verteilung erreicht werden.

Wird der Auslaßkanal 13 wie im vorliegenden Fall nahe der Drehachse oder auf der Drehachse liegend angebracht, so bildet sich in diesem Bereich ein immer schwächeres Schwerefeld aus. Die Schichtung der zu trennenden Flüssigkeit wird in diesem Bereich aufgehoben. Zur weitgehenden Vermeidung von Vermischungen zweier zu trennender Bestandteile kann der Bereich zwischen der äußeren Schale 2 und der inneren Begrenzung 9 im oberen Bereich bis zu einer Entfernung von ca. 2 cm vom Mittelpunkt entfernt sehr schmal ausgeführt werden. So kommt es stehts nur bei einer sehr geringen Menge Flüssigkeit zu einer Entmischung.

Nachdem der fraktionierte Bestandteil aus dem Separationsraum 10 entleert ist, wird die Pumpe 18 in ihrer Pumprichtung wieder umgekehrt, das Absperrventil 20 geöffnet und erneut zu separierende Flüssigkeit aus dem Vorratsbehälter 17 über den Zulaufkanal 11 dem Separationsraum 10 zugeführt.

Die einzelnen Vorgänge, d.h. das Entleeren des Separationsraums 10 und das anschließende Zentrifugieren, bis sich wieder in dem Separationsraum 10 eine ausreichende Menge des separierten Bestandteils angesammelt hat, kann beliebig oft wiederholt werden, ohne dabei die Drehung des Zentrifugierbehälters 2 anzuhalten. Hierdurch ergibt sich eine große Zeitersparnis, da keine Stillstandszeiten zwischen den einzelnen Entleerungen des Separationsraums eingeplant werden müssen, sondern die Separation auch großer Mengen an zu separierender Flüssigkeit im quasi-kontinuierlichen Betrieb erfolgt.

Neben dem Fraktionsbehälter 36 können weitere, nicht dargestellte Fraktionsbehälter vorgesehen werden, um aus den zu separierenden Flüssigkeiten weitere Bestandteile heraus zu fraktionieren. In einem solchen Fall würde, nachdem die gesamte oder eine vorgegebene Menge an Flüssigkeit aus dem Vorratsbehälter 17 separiert ist, die sich in dem Sammelbehälter 29 befindliche Flüssigkeit erneut dem Separationsraum 10 zugeführt werden, um dann einen weiteren Bestandteil aus dieser, von der ersten Fraktion befreiten Flüssigkeit, zu separieren. Hierzu kann die Verfahrensweise entsprechend derjenigen, die vorstehend beschrieben ist, erfolgen, d.h. es kann auch im kontinuierlichen Betrieb ohne die Zentrifuge anzuhalten verfahren werden.

Mit der Anordnung gemäß Figur 1 können beim Separieren von Blut zum Beispiel gezielt Thrombozyten in der Glocke 2 angereichert werden, indem der Separationsraum 10 der Zentrifugierglocke 2 mit Blut aus dem Vorratsbehälter 17 oder unmittelbar von dem Spender solange befüllt wird, bis der Separationsraum 10 mit den zellulären Bestandteilen fast völlig gefüllt ist. Die Thrombozyten reichern sich dabei in der Mitte des Separationsraums 10 an. Bei diesem ersten Befüllen wird die sich in dem Separationsraum 10 befindliche Flüssigkeit in einen an den Ablaufkanal 13 angeschlossenen Beutel 29 oder 46 geleitet. Nach weiterer Befüllung der Zentrifugierglocke 2 bzw. des Separationsraums 10 tritt Plasma über den seitlichen Stutzen 14 aus und wird ebenfalls in einem der Beutel 29 oder 46 gesammelt. Falls bei dieser Verfahrensweise das Plasma das gewünschte Zielprodukt ist, wird es in dem Beutel 29 gesammelt, während die Luft in den Beutel 46 überströmt. Werden zum Beispiel Thrombozyten gewünscht, wird man Luft und Plasma im Beutel 46 sammeln.

Dann werden, bei sich drehender Zentrifugierglocke, die Erythrozyten abgepumpt (ggf. kann ausgangsseitig, d.h. im Anschluß an den seitlichen Stutzen 14, eine zusätzliche, nicht dargestellte Pumpe vorgesehen werden). Das dabei entstehende Volumendefizit wird wieder mit zuvor übergelaufenem Plasma aus dem Beutel 46 gefüllt. Die Zentrifugierglocke 2 bleibt somit immer gefüllt. Am Ende dieses Zyklus hat man somit alle Thrombozyten des ersten Zyklus sowie Plasma in der Zentrifugierglocke 2 bzw. dem Separationsraum 10. Der nächste Zyklus erfolgt analog zu dem ersten. Danach erhält man die Thrombozyten von zwei Zyklen in der Zentrifugierglocke 2. Wenn dann genug Thrombozyten in der Zentrifugierglocke 2, nach einer entsprechenden Anzahl von Zyklen, angereichert sind, überfüllt man die Glocke mit den Zellen und leitet so die Thromobzyten in einem Vorgang in den Sammelbeutel bzw. das -gefäß 29. Hierzu wird ein Ventil 44, das dem Beutel 46 zugeordnet ist, geschlossen und ein Ventil 43, das dem Beutel 29 zugeordnet ist, wird geöffnet. Über einen Sensor 45 kann zusätzlich die Grenze zwischen den einzelnen Kompartimenten kontrolliert werden. Erst am Ende des Verfahrensablaufs, wie er vorstehend beschrieben ist, wird die Zentrifugierglocke 2 angehalten und der Inhalt dem Spender zurückgegeben. Im stehenden Zustand wird dann das Volumendifizit in der Zentrifugierglocke 2 nicht durch Plasma, sondern durch Luft aus dem Beutel 46, ausgeglichen. Analog zu der beschriebenen Verfahrensweise kann man auch bei der Separation von Blut nur die äußersten, saubersten Erythrozyten abpumpen.

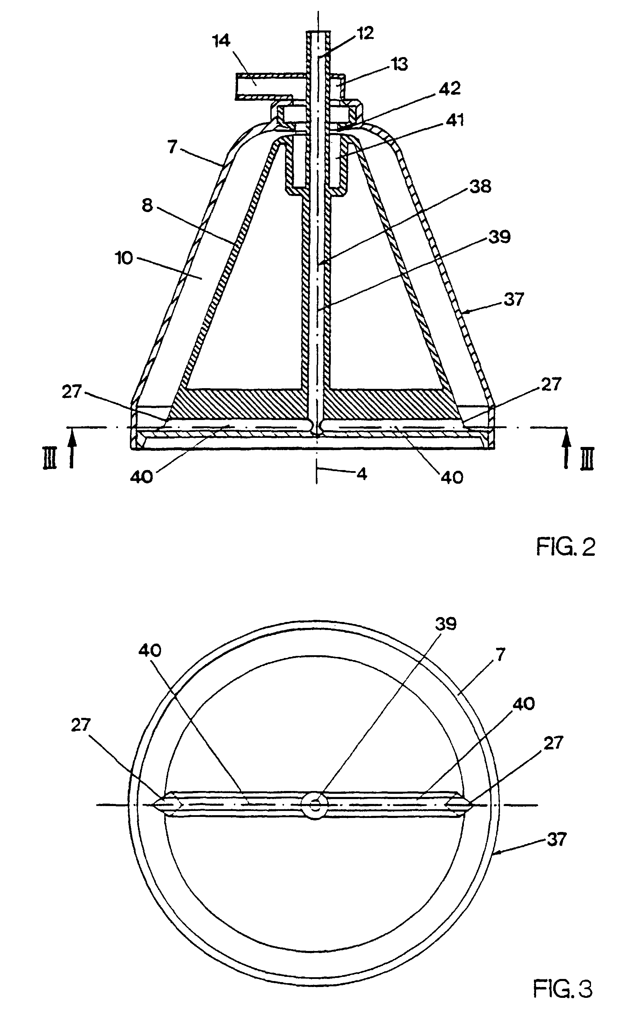

Zwei weitere Ausführungsformen eines Zentrifugierbehälters bzw. einer Zentrifugierglokke sind in den Figuren 2 und 3 sowie den Figuren 4 und 5 dargestellt.

Die Zentrifugierglocken gemäß den Figuren 2 bis 5 entsprechen in ihrer äußeren Form etwa dem Zentrifugierbehälter 2, wie er in Figur 1 dargestellt ist. Soweit in den Figuren 2 bis 5 Teile mit denjenigen der Ausführungsform der Figur 1 vergleichbar sind, wurden für solche Teile die entsprechenden Bezugszeichen verwendet, die auch in Figur 1 verwendet sind.

Wie ein Vergleich der Figur 1 und der Figur 2 zeigt, umfaßt der Zentrifugierbehälter 37 der Figur 2 nicht das Einsatzteil 9, das, parallel zu dem Separationsraum 10, auf der Innenseite den Zufuhrkanal 11 abteilt. Vielmehr ist in der Ausführungsform der Figur 2 der Zufuhrkanal 38 in zwei Abschnitte, einen ersten Abschnitt 39 und einen zweiten Abschnitt 40, unterteilt. Der erste Abschnitt 39 verläuft in Verlängerung der Drehachse 4 bis zu dem Boden des Zentrifugierbehälters 2, wo der erste Abschnitt 39 des Zufuhrkanals 38 dann in zwei radial gegenüberliegend verlaufende zweite Abschnitte 40 übergeht, wie anhand der Figur 3 gut zu erkennen ist. Diese radialen, zweiten Abschnitte 40 gehen dann an der Verbindungsstelle 27, die mit der Verbindungsstelle 27 der Ausführungsform der Figur 1 vergleichbar ist, in den Separationsraum 10 über. Diese Anordnung des Zulaufkanals 10 hat gegenüber der Anordnung des Zulaufkanals 10 der Figur 1 den Vorteil, daß das die innere Behälterwand bildende Teil und der Zufuhrkanal 38 aus einem Teil gefertigt werden können. Es ist anzumerken, daß in Figur 2 der Einlaß 12 und der Kanal 38 bzw. 39 über eine nicht dargestellte Dichtung, die in dem Raum 41 angeordnet wird, getrennt sind.

Im oberen Bereich des Zentrifugierbehälters 2 der Figur 2 ist ein erweiterter Bereich 41 vorgesehen, in den Übergangsöffnungen 42 münden, die den Separationsraum 10 mit diesem erweiternden Bereich 41 und dem Ablaufkanal und den seitlichen Stutzen 14 verbinden. Dieser erweiterte Bereich 41 dient zur Aufnahme einer unteren, nicht dargestellten Dichtung.

Der Separationsraum 10 der Zentrifugierbehälter 2 und 37 der Figuren 1 und 2 sollte, je nach Anwendung, ein Volumen zwischen 75 und 350 ml haben.

Der Zentrifugierbehälter 2 entsprechend der Ausführungsform gemäß den Figuren 2 und 3 kann in die Zentrifugiereinheit 1, wie sie in Figur 1 gezeigt ist, anstelle des dort gezeigten Zentrifugierbehälters 2 eingesetzt werden. Die Verfahrensweise erfolgt dann entsprechend derjenigen, die zuvor anhand der Figur 1 erläutert worden ist.

Wie bereits eingangs erwähnt ist, ist es mit den in den Figuren dargestellten bzw. Zentrifugierbehältern möglich, auch eine Befüllung bei voller Betriebsdrehzahl vorzunehmen. Diese Möglichkeit ist insbesondere dadurch gegeben, daß die Öffnung des Auslasses 13 direkt an der Wand des Einlasses 12 beginnt, da diese koaxial zueinander angeordnet sind. Nur wenn der Zentrifugierbehälter vollständig mit Flüssigkeit, insbesondere zu Beginn des Zentrifugiervorgangs, gefüllt werden kann, ist gewährleistet, daß sich in der Trennglocke keine Druckdifferenzen bilden, was ein Absaugen der Flüssigkeit ansonsten praktisch unmöglich machen würde. Die vollständige Füllung des Zentrifugierbehälters mit Flüssigkeiten ist erforderlich, um ein Absaugen bei voller Betriebsdrehzahl zu ermöglichen. Eine solche vollständige Befüllung des Zentrifugierbehälters mit Flüssigkeit ist gerade durch die spezielle Anordnung des Zulaufs und des Ablaufs unmittelbar im Bereich der Achse 4 des Zentrifugierbehälters gegeben. Während in der Ausführungsform, wie sie in Figur 2 dargestellt ist, der Separationsraum 10 in seinem oberen Bereich im Querschnitt verringert ist und unmittelbar in den Ablaufkanal 13 mündet, wobei die Querschnittsdimension dieses Übergangs 47, in Richtung der Drehachse 4 gesehen, etwa 2 mm beträgt, ist in der Ausführungsform der Figur 4 dieser Kanal 47 noch ausgeprägter gestaltet, indem er eine Länge von etwa 30 mm in der radialen Richtung der Drehachse 4 besitzt. Die Ausführungsform, wie sie in den Figuren 4 und 5 dargestellt ist, entspricht in ihrem grundsätzlichen Aufbau, insbesondere in Bezug auf den Zufuhrkanal 38 bzw. den ersten Abschnitt 39 und den zweiten Abschnitt 40, der Ausführungsform gemäß Figur 2. Im Gegensatz zu der Ausführungsform der Figur 2 ist allerdings die Verbindungsstelle 27 zwischen dem zweiten Abschnitt 40 und dem Separationsraum weiter radial nach außen bis annähernd zu der äußeren Behälterwand 7 hin verlegt, so daß zwischen der Austrittsstelle aus dem jeweiligen zweiten Abschnitt 40 und der Innenseite der äußeren Behälterwand 7 nur ein Abstand von einigen Millimetern verbleibt. Hierdurch ist gewährleistet, daß auch die äußeren Schichten von den schweren, separierten Bestandteilen, die sich in diesem Bereich ansammeln, abgeführt werden können. Weiterhin sind Ablenkteile 48, die beispielsweise schaufelartig ausgebildet sind, wie in Figur 5 gezeigt ist, vorgesehen, um die zugeführte, aus dem Abschnitt 40 in den Separationsraum 10 einströmende Flüssigkeit zu verwirbeln. Weiterhin ist bei der Ausführungsform 4 der Zentrifugierbehälter 7, um den Zufuhrkanal 38 in einfacher Weise auszubilden, mit einem Boden 49 verschlossen, dem ein unteres Einsatzteil 50 zugeordnet ist, das einen Teil der zweiten Abschnitt 40 des Zufuhrkanals 38 bildet bzw. begrenzt. In einem weiteren Einsatzteil 51 ist der Zufuhrkanal 38 eingebracht, darüberhinaus verbindet dieses Einsatzteil 51 die innere Behälterwand 8, die den Separationsraum 10 begrenzt.

Wie die Figur 6 zeigt, ist insbesondere mit einer Ausbildung der Kanäle entsprechend der Ausführungsform der Figur 4 gewährleistet, daß nur geringe Drücke überwunden werden müssen, und zwar aufgrund des geringen Abstands des Übergangs des Separationsraums 10 in den Ablaufkanal 13, um die separierten Bestandteile bei voller Drehzahl abzusaugen, wobei in Figur 6 fünf Kurven dargestellt sind, die sich auf unterschiedliche Drehzahlen U/min (rpm) beziehen, wobei die unterste Kurve der geringsten Drehzahl (4.000 U/min) zugeordnet ist, während die oberste Kurve der höchsten Drehzahl (6.000 U/min) zugeordnet ist.

## Patentansprüche

1. Verfahren zum Betreiben einer Zentrifugiereinheit (1) mit einem Zentrifugierbehälter (2) oder mit einer Zentrifugierglocke (2), zum Separieren von Bestandteilen aus einer Bestandteile mit unterschiedlichem, spezifischen Gewicht enthaltenden Flüssigkeit aufweist, wobei der Zentrifugierbehälter (2) einen Separationsraum (10), einen Zulauf (12) für die Flüssigkeit zu dem Separationsraum (10) und einen Ablauf (13, 14) für einen separierten Bestandteil aus dem Separationsraum (10) aufweist, wobei der Zulauf (12) und der Ablauf (13, 14) durch eine Drehdurchführung am oberen Ende des Zentrifugierbehälters hindurchgeführt sind und wobei der Zulauf (12) mit einem Zufuhrkanal (11) in Verbindung steht, der in einem definierten Bereich des Zentrifugierbehälter (2) in den Separationsraum (10) mündet, wobei über den Zulauf (12) die zu zentrifugierende Flüssigkeit zugeführt wird, **dadurch gekennzeichnet, daß** eine Pumpeinrichtung (18) zum Befüllen und Entleeren des Zentrifugierbehälters (2) vorgesehen ist, wobei nach dem Separieren einer erwünschten Menge unter weiterem, kontinuierlichen Rotieren des Zentrifugierbehälters (2) bei voller Betriebsdrehzahl die Strömungsrichtung zwischen Zulauf (12) und Ablauf (13, 14) umgekehrt wird, so daß über den Zufuhrkanal (11) und den Zulauf (12) aus dem Separationsraum (10) zumindest einer der separierten Bestandteile abgeführt wird, wobei danach die Strömungsrichtung wieder umgekehrt wird, so daß dann über den Zulauf (12) erneut zu separierende Flüssigkeit zugeführt wird, wobei bei dem Abführen der separierten Bestandteile über den mit dem Separationsraum (10) in Verbindung stehenden Ablauf (13, 14) eine Ausgleichsflüssigkeit zugeführt wird.

2. Verfahren zum Betreiben einer Zentrifugiereinheit nach Anspruch 1, **dadurch gekennzeichnet, daß** die Flüssigkeit über einen Zufuhrkanal (11) zugeführt wird, der im unteren Bereich des Zentrifugierbehälters (2) in den Separationsraum (10) mündet, und nach Umkehr der Strömungsrichtung über den Zufuhrkanal (11) separierte Bestandteile mit höherem spezifischen Gewicht abgeführt werden.

3. Verfahren nach Anspruch 1, **dadurch gekennzeichnet, daß** die Umschaltung der Pumpeinrichtung (18) und damit der Strömungsrichtung zwischen Zulauf (12) und Ablauf (13, 14) durch eine Sensoreinrichtung (31) ausgelöst wird, die eine bestimmte, separierte Menge der schweren Bestandteile in dem Separationsraum (10) erfaßt.

4. Verfahren nach Anspruch 3, **dadurch gekennzeichnet, daß** die Erfassung des Füllstands optisch erfolgt.

5. Verfahren nach Anspruch 1, **dadurch gekennzeichnet, daß** als Ausgleichsflüssigkeit eine solche mit einem gegenüber dem separierten Bestandteil/den separierten Bestandteilen geringen spezifischen Gewicht zugeführt wird.

6. Verfahren nach Anspruch 5, **dadurch gekennzeichnet, daß** als Ausgleichsflüssigkeit zu separierende Flüssigkeit zugeführt wird.

7. Verfahren nach Anspruch 1, **dadurch gekennzeichnet, daß** die zu separierende Flüssigkeit bei dem Einströmen in den Separationsraum (10) verwirbelt wird.

8. Zentrifugiereinheit (1) mit einem Zentrifugierbehälter oder mit einer Zentrifugierglocke (2) zum Separieren von Bestandteilen aus einer Bestandteile mit unterschiedlichem, spezifischen Gewicht enthaltenden Flüssigkeit wobei der Zentrifugierbehälter (2) einen Separationsraum (10), einen Zulauf (12) für die Flüssigkeit zu dem Separationsraum (10) und einen Ablauf (13, 14) für einen separierten Bestandteil aus dem Separationsraum (10) aufweist, wobei der Zulauf (12) und der Ablauf (13, 14) durch eine Drehdurchführung am oberen Ende des Zentrifugierbehälters hindurchgeführt sind und wobei der Zulauf (12) mit einem Zufuhrkanal (11) in Verbindung steht, der am unteren Bereich des Zentrifugierbehälters in den Separationsraum (10) mündet, wobei der Zulauf (12), der Zufuhrkanal (11; 38), der Separationsraum (10) und der Ablauf (13, 14) in Strömungsrichtung gesehen aufeinanderfolgende, voneinander getrennte Strömungswege bilden, **dadurch gekennzeichnet, daß** eine Pumpeinrichtung (18) zum Befüllen und Entleeren des Zentrifugierbehälters (2), vorgesehen ist, und daß die Pumpeinrichtung (18), die Strömungsrichtung zwischen Zulauf und Ablauf umkehrend, umschaltbar ist zum Abführen, dann über den Zufuhrkanal (18; 38) und den Zulauf (12), eines separierten Bestandteils, wobei der Übergang zwischen dem Separationsraum (10) und dem Ablauf (14) nahe der Rotationsachse, maximal 10 mm von dieser beabstandet, angeordnet ist.

9. Zentrifugiereinheit nach Anspruch 8, **dadurch gekennzeichnet, daß** der Abstand zwischen dem Querschnitt des Ablaufs (13, 14) und Rotationsachse (4) zwischen 2 bis 6 mm beträgt.

10. Zentrifugiereinrichtung nach Anspruch 8, **dadurch gekennzeichnet, daß** der Zulaufkanal (11; 38) im unteren Bereich des Zentrifugierbehälters (2) mündet (27) und zur Abfuhr separierter Bestandteile mit höherem spezifischen Gewicht dient.

11. Zentrifugiereinheit nach Anspruch 8, **dadurch gekennzeichnet, daß** der Zufuhrkanal (38) mindestens in zwei Abschnitte (39, 40) unterteilt ist, wobei der erste Abschnitt (38) mit seiner Achse entlang der Rotationsachse (4) des Zentrifugierbehälters (2) verläuft.

12. Zentrifugiereinheit nach Anspruch 11, **dadurch gekennzeichnet, daß** der zweite Teil des Zufuhrkanals (38) im wesentlichen in radialer Richtung zu der Achse des Zentrifugierbehälters (2) verläuft.

13. Zentrifugiereinheit nach Anspruch 11, **dadurch gekennzeichnet, daß** der erste Abschnitt (40) des Zufuhrkanals (38) in mehrere radial verlaufende zweite Abschnitte des Zufuhrkanals (38) übergeht.

14. Zentrifugiereinheit nach Anspruch 13, **dadurch gekennzeichnet, daß** die Zufuhrkanäle (38, 40) in radialer Richtung gesehen gleichmäßig verteilt sind.

15. Zentrifugiereinheit nach einem der Ansprüche 8 bis 14, **dadurch gekennzeichnet, daß** der Zufuhrkanal (11; 38) einen wesentlich geringeren Strömungsquerschnitt aufweist als der Strömungsquerschnitt des Separationsraums (10).

16. Zentrifugiereinheit nach Anspruch 11, **dadurch gekennzeichnet, daß** der erste Abschnitt des Zufuhrkanals (11) nahe zu dem Zulauf (12) radial zu der Rotationsachse (4) des Zentrifugierbehälters (2) verläuft, während sich der zweite Abschnitt im wesentlichen in Richtung der Rotationsachse (4) verlaufend an den ersten Abschnitt anschließt.

17. Zentrifugiereinheit nach Anspruch 16, **dadurch gekennzeichnet, daß** der Zufuhrkanal (11) und der Separationsraum (10) voneinander durch eine gemeinsame Trennwand (9) getrennt sind.

18. Zentrifugiereinheit nach Anspruch 17, **dadurch gekennzeichnet, daß** der Separationsraum (10) und der Zufuhrkanal (11) zu der Drehachse (4) des Zentrifugierbehälters (2) rotationssymmetrische Ringräume bilden.

19. Zentrifugiereinheit nach einem der Ansprüche 8 bis 18, **dadurch gekennzeichnet, daß** der Separationsraum (10) in seiner Erstreckung in Strömungsrichtung zu der Drehachse (4) geneigt verläuft, wobei das zu dem Ablauf (13) hin liegende Ende näher zu der Drehachse (4) als der Übergang (27) zwischen Zufuhrkanal (11) und Separationsraum (10) liegt.

20. Zentrifugiereinheit nach einem der Ansprüche 8 bis 19, **dadurch gekennzeichnet, daß** die den Zufuhrkanal (11) und den Separationsraum (10) begrenzenden Wände (7, 8, 9) durch becherförmige Teile mit unterschiedlichen Radien zur Drehachse (4) gebildet sind.

21. Zentrifugiereinheit nach einem der Ansprüche 8 bis 20, **dadurch gekennzeichnet, daß** dem Separationsraum (10) eine Sensoreinrichtung (31) zur Erfassung des Fülllstands der separierten Fraktion der Bestandteile mit dem hohen spezifischen Gewicht zugeordnet ist.

22. Zentrifugiereinheit nach Anspruch 21, **dadurch gekennzeichnet, daß** die Sensoreinrichtung (31) nahe dem Ablauf (13) positioniert ist.

23. Zentrifugiereinheit nach Anspruch 8, **dadurch gekennzeichnet, daß** im Übergangsbereich (27) zwischen Zufuhrkanal (11) und Separationsraum (10) die Strömung verwirbelnde Ablenkteile eingesetzt sind.

## Claims

1. Process for operating a centrifuging unit (1), which includes a centrifuging container (2) or a centrifuging bowl (2), for separating the components of a liquid containing components with different specific weights, wherein the centrifuging container (2) containing a separation chamber (10), an inlet (12) for the liquid to the separation chamber (10), and an outlet (13, 14) for a separated component from the separation chamber (10), with the inlet (12) and outlet (13, 14) extending through a revolving passage at the upper end of the centrifuging bowl, with the inlet (12) being in communication with an supply channel (11) which empties in a defined area of the centrifuging bowl (2) into the separation chamber (10), and wherein liquid to be centrifuged being supplied via the inlet, **characterized in that** a pump device (18) to fill and empty the centrifuging bowl (2) is provided, whereby after separation of a desired quantity while, rotation of the centrifuging container continues at full operation speed, the direction of flow is reversed between inlet (12) and outlet (13, 14), so that via the supply channel (11) and the inlet (12) from the separation chamber (10) at least one of the separated components is drawn off, whereby thereafter the direction of flow is reversed again, so that via the inlet (12), liquid to be separated are again supplied, whereby in drawing off separated components via the outlet (13, 14) that connects with the separation chamber, a compensating liquid is supplied.

2. Process for operating a centrifuging unit according to claim 1, **characterized in that** the liquid is supplied via an inlet channel (11) that empties in the lower area of the centrifuging bowl (2) into the separation chamber (10), and after the direction of flow is reversed, separated components with higher specific weights are drawn off via the supply channel (11).

3. Process according to claim 1, **characterized in that** switchover the pump device (18) and thus of the direction of flow between the inlet (12) and outlet (13, 14) is triggered by a sensor device (31), which detects a certain separated quantity of the heavy components in the separation chamber (10).

4. Process according to claim 3, **characterized in that** detection of the filling level is done optically.

5. Process according to claim 1, **characterized in that** the compensating liquid is one that has a small specific weight compared to the separated component/the separated components.

6. Process according to claim 5, **characterized in that** the liquid to be separated is supplied as a compensating liquid.

7. Process according to claim 1, **characterized in that** the liquid to be separated is caused to be turbulent as it flows into the separation chamber (10).

8. Centrifuging unit (1), with a centrifuging container or a centrifuging bowl (2), for separating components of a liquid containing components with different specific weights, wherein with the centrifuging container (2) containing a separation chamber (10), an inlet (12) for the liquid to the separation chamber (10), and an outlet (13, 14) for a separated component from the separation chamber (10), whereby the inlet (12) and outlet (13, 14) extend through a revolving passage at the upper end of the centrifuging container; and whereby the inlet (12) is connected to an supply channel (11) which empties in the lower area of the centrifuging container into the separation chamber (10), wherein the inlet (12), the supply channel (11; 38), the separation chamber (10), and the outlet (13, 14), seen in the direction of flow, form flow paths that follow each other and are separated from each other, **characterized in that** a pump device (18) to fill and empty the centrifuging container (2) is provided and that the pump device (18), reversing the direction of flow between inlet and outlet, can be switched over to withdraw a separated component then via the supply channel (18; 38) and the inlet (12), whereby the transition between the separation chamber (10) and the outlet (14) is situated close to the rotational axis, at a maximum distance of 10 mm away from it.

9. Centrifuging unit according to claim 8, **characterized in that** the distance between the cross section of the outlet (13, 14) and rotational axis (4) is between 2 and 6 mm.

10. Centrifuging unit according to claim 8, **characterized in that** the inlet channel (11; 38) empties (27) into the lower area of centrifuging container (2), and serves for removal of separated components with a high specific weight.

11. Centrifuging unit according to claim 8, **characterized in that** the feed channel (38) is divided into at least two sections (39, 40), with the first section (38) with its axis running along the rotational axis (4) of centrifugal container (2).

12. Centrifuging unit according to claim 11, **characterized in that** the second part of the supply channel (38) runs essentially in a direction radial to the axis of the centrifuging container (2).

13. Centrifuging unit according to claim 11, **characterized in that** the first section (40) of supply channel (38) makes a transition into several radially-running second sections of supply channel (38).

14. Centrifuging unit according to claim 13, **characterized in that** the supply channels (38, 40), seen in a radial direction, are uniformly divided.

15. Centrifuging unit according to one of claims 8 to 14, **characterized in that** the supply channel (11; 38) has a flow cross section that is considerably smaller than the flow cross section of separation chamber (10).

16. Centrifuging unit according to claim 11, **characterized in that** the first section of feed channel (11), close to inlet (12) runs radial to the rotational axis (4) of centrifuging container (2), while the second section essentially runs in the direction of rotational axis (4) as it connects to the first section.

17. Centrifuging unit according to claim 16, **characterized in that** the supply channel (11) and the separation chamber (10) are separated from each other by a common separating wall (9).

18. Centrifuging unit according to claim 17, **characterized in that** the separation chamber (10) and the supply channel (11) form annular spaces that are rotationally symmetric to the rotational axis (4) of centrifuging container (2).

19. Centrifuging unit according to one of claims 8 to 18, **characterized in that** the separation chamber (10), in its extension in the direction of flow runs inclined to rotational axis (4), whereby the end that is toward the outlet (13) is closer to the rotational axis (4) and the transition (27) between supply channel (11) and separation chamber (10).

20. Centrifuging unit according to one of claims 8 to 19, **characterized in that** the walls (7, 8, 9) separating supply channel (11) and separation chamber (10) are formed by beaker-shaped pieces with radii to rotational axis (4) which vary.

21. Centrifuging unit according to one of claims 8 to 20, **characterized in that** a sensor device (31) is assigned to the separation chamber (10) for detection of the filling level of the separated fraction of components with high specific weights.

22. Centrifuging unit according to claim 21, **characterized in that** the sensor device (31) is positioned near the outlet (13).

23. Centrifuging unit according to claim 8, **characterized in that** the transitional area (27) between supply channel (11) and the separation chamber (10), diversion pieces are inserted that impart turbulence to the flow.

## Revendications

1. Procédé d'exploitation d'une unité de centrifugation (1) avec un réservoir de centrifugation (2) ou avec une cloche de centrifugation (2), pour séparer des composants d'un liquide contenant des composants d'un poids spécifique différent, où le réservoir de centrifugation (2) présente une enceinte de séparation (10), une amenée (12) du liquide vers l'enceinte de séparation (10) et une évacuation (13, 14) pour un composant séparé de l'enceinte de séparation (10), où l'amenée (12) et l'évacuation (13, 14) sont amenées à passer à travers un passage tournant à l'extrémité supérieure du réservoir de centrifugation, et où l'amenée (12) est en liaison avec un canal d'amenée (11) qui débouche dans une zone définie du réservoir de centrifugation (2) dans l'enceinte de séparation (10), où est acheminé par l'amenée (12) le liquide à centrifuger, **caractérisé en ce qu'**il est prévu une installation de pompe (18) pour le remplissage et le vidage du réservoir de centrifugation (2), où après la séparation d'une quantité souhaitée, par une rotation continuelle ultérieure du réservoir de centrifugation (2), à pleine vitesse de régime, la direction d'écoulement entre l'amenée (12) et l'évacuation (13, 14) est inversée de telle sorte que par le canal d'amenée (11) et l'amenée (12), au moins l'un des composants séparés est évacué de l'enceinte de séparation (10) et où ensuite la direction d'écoulement est de nouveau inversée de telle sorte que par l'amenée (12), de nouveau du liquide à séparer est acheminé, où lors de l'évacuation des composants séparés par l'évacuation (13, 14) en liaison avec l'enceinte de séparation (10), un liquide de compensation est acheminé.

2. Procédé d'exploitation d'une unité de centrifugation selon la revendication 1, **caractérisé en ce que** le liquide est acheminé par un canal d'amenée (11) qui débouche dans la zone inférieure du réservoir de centrifugation (2) dans l'enceinte de séparation (10) et, après l'inversion de la direction d'écoulement, des composants séparés d'un poids spécifique plus élevé sont évacués par le canal d'amenée (11).

3. Procédé selon la revendication 1, **caractérisé en ce que** la commutation de l'installation de pompe (18) et donc de la direction d'écoulement entre l'amenée (12) et l'évacuation (13, 14) est déclenchée par une installation à détecteur (31) qui détecte une quantité séparée déterminée des composants lourds dans l'enceinte de séparation (10).

4. Procédé selon la revendication 3, **caractérisé en ce que** la détection du niveau de remplissage a lieu d'une manière optique.

5. Procédé selon la revendication 1, **caractérisé en ce que**, comme liquide de compensation, un liquide d'un poids spécifique plus bas par rapport au composant séparé / composants séparés est amené.

6. Procédé selon la revendication 5, **caractérisé en ce que** comme liquide de compensation, du liquide à séparer est acheminé.

7. Procédé selon la revendication 1, **caractérisé en ce que** le liquide à séparer est mis en tourbillonnement lors de l'afflux dans l'enceinte de séparation (10).

8. Unité de centrifugation (1) avec un réservoir de centrifugation ou avec une cloche de centrifugation (2) pour séparer des composants d'un liquide contenant des composants d'un poids spécifique différent, où le réservoir de centrifugation (2) présente une enceinte de séparation (10), une amenée (12) du liquide à l'enceinte de séparation (10) et une évacuation (13, 14) pour un composant séparé de l'enceinte de séparation (10), où l'amenée (12) et l'évacuation (13, 14) sont amenés à passer à travers un passage tournant à l'extrémité supérieure du réservoir de centrifugation et où l'amenée (12) est en liaison avec un canal d'amenée (11) qui débouche dans la zone inférieure du réservoir de centrifugation dans l'enceinte de séparation (10), où l'amenée (12), le canal d'amenée (11 ; 38), l'enceinte de séparation (10) et l'évacuation (13, 14), en regardant dans la direction d'écoulement, forment des chemins d'écoulement successifs, séparés les uns des autres, **caractérisée en ce qu'**il est prévu une unité de pompe (18) pour remplir et vider le réservoir de centrifugation (2), et **en ce que** l'installation de pompe (18) peut être commutée, en inversant la direction d'écoulement entre l'amenée et l'évacuation, pour l'évacuation, alors par le canal d'amenée (18 ; 38) et l'amenée (12), d'un composant séparé, où le passage entre l'enceinte de séparation (10) et l'évacuation (14) est disposé à proximité de l'axe de rotation, au maximum à une distance de 10mm de celui-ci.

9. Unité de centrifugation selon la revendication 8, **caractérisée en ce que** l'écart entre la section transversale de l'évacuation (13, 14) et l'axe de rotation (4) représente entre 2 et 6 mm.

10. Unité de centrifugation selon la revendication 8, **caractérisée en ce que** le canal d'amenée (11 ; 38) débouche (27) dans la zone inférieure du réservoir de centrifugation (2) et sert à l'évacuation de composants séparés d'un poids spécifique plus élevé.

11. Unité de centrifugation selon la revendication 8, **caractérisée en ce que** le canal d'amenée (38) est divisé en au moins deux tronçons (39, 40), où le premier tronçon (38) s'étend avec son axe le long de l'axe de rotation (4) du réservoir de centrifugation (2).

12. Unité de centrifugation selon la revendication 11, **caractérisée en ce que** la deuxième partie du canal d'amenée (32) s'étend sensiblement dans la direction radiale à l'axe du réservoir de centrifugation (2).

13. Unité de centrifugation selon la revendication 11, **caractérisée en ce que** le premier tronçon (40) du canal d'amenée (38) rejoint plusieurs seconds tronçons s'étendant radialement du canal d'amenée (38).

14. Unité de centrifugation selon la revendication 13, **caractérisée en ce que** les canaux d'amenée (38, 40) sont répartis uniformément en regardant dans la direction radiale.

15. Unité de centrifugation selon l'une des revendications 8 à 14, **caractérisée en ce que** le canal d'amenée (11 ; 38) présente une section transversale d'écoulement sensiblement plus réduite que la section transversale d'écoulement de l'enceinte de séparation (10).

16. Unité de centrifugation selon la revendication 11, **caractérisée en ce que** le premier tronçon du canal d'amenée (11) s'étend à proximité de l'amenée (12) radialement à l'axe de rotation (4) du réservoir de centrifugation (2) tandis que le second tronçon fait suite au premier tronçon en s'étendant sensiblement en direction de l'axe de rotation (4).

17. Unité de centrifugation selon la revendication 16, **caractérisée en ce que** le canal d'amenée (11) et l'enceinte de séparation (10) sont séparés l'un de l'autre par une paroi de séparation commune (9).

18. Unité de centrifugation selon la revendication 17, **caractérisée en ce que** l'enceinte de séparation (10) et le canal d'amenée (11) forment des enceintes annulaires symétriques en rotation à l'axe de rotation (4) du réservoir de centrifugation (2).

19. Unité de centrifugation selon l'une des revendications 8 à 18, **caractérisée en ce que** l'enceinte de séparation (10) dans son extension dans la direction d'écoulement, s'étend selon une inclinaison à l'axe de rotation (4), où l'extrémité située vers l'évacuation (13) est plus proche de l'axe de rotation (4) que le passage (27) entre le canal d'amenée (11) et l'enceinte de séparation (10).

20. Unité de centrifugation selon l'une des revendications 8 à 19, **caractérisée en ce que** les parois (7, 8, 9) délimitant le canal d'amenée (11) et l'enceinte de séparation (10) sont formées par des parties en forme de pot avec des rayons différents à l'axe de rotation (4).

21. Unité de centrifugation selon l'une des revendications 8 à 20, **caractérisée en ce qu'**il est associé à l'enceinte de séparation (10) une installation à détecteur (31) pour la détection du niveau de remplissage de la fraction séparée des composants présentant le poids spécifique élevé.

22. Unité de centrifugation selon la revendication 21, **caractérisée en ce que** l'installation à détecteur (31) est positionnée à proximité de l'évacuation (13).

23. Unité de centrifugation selon la revendication 8, **caractérisée en ce que** des parties de déviation provoquant le tourbillonnement de l'écoulement sont placées dans la zone de transition (27) entre le canal d'amenée (11) et l'enceinte de séparation (10).
